(19) Europäisches Patentamt
European Patent Office
Office européen des brevets

(11) Veröffentlichungsnummer: **0 241 690 A2**

(12) **EUROPÄISCHE PATENTANMELDUNG**

(21) Anmeldenummer: **87103026.8**

(22) Anmeldetag: **04.03.87**

(51) Int. Cl.⁴: **C 07 C 119/042,** C 08 G 18/73

(30) Priorität: **13.03.86 DE 3608354**
**21.06.86 DE 3620821**

(43) Veröffentlichungstag der Anmeldung: **21.10.87**
**Patentblatt 87/43**

(84) Benannte Vertragsstaaten: **AT BE CH DE ES FR GB IT LI NL SE**

(71) Anmelder: **BAYER AG, Konzernverwaltung RP Patentabteilung, D-5090 Leverkusen 1 Bayerwerk (DE)**

(72) Erfinder: **Heitkämper, Peter, Dr., Fliederweg 14, D-4047 Dormagen 11 (DE)**
Erfinder: **Klein, Gerhard, Dr., von-Flotow-Strasse 7, D-4019 Monheim (DE)**
Erfinder: **Arlt, Dieter, Prof.Dr., Rybniker Strasse 2, D-5000 Köln 80 (DE)**

(54) **Aliphatische Diisocyanate und ihre Verwendung zur Herstellung von Polyurethankunststoffen.**

㊼ Diisocyanate der allgemeinen Formel

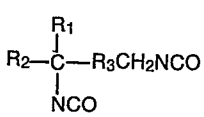

I

in welcher

$R_1$ und $R_2$ für gleiche oder verschiedene Reste stehen und Alkylgruppen mit 1–4 Kohlenstoffatomen bedeuten und

$R_3$ für einen zweiwertigen, gegebenenfalls verzweigten, gesättigten aliphatischen Kohlenwasserstoffrest mit 2–9 Kohlenstoffatomen steht,

und ihre Verwendung als Isocyanatkomponente bei der Herstellung von Polyurethankunststoffen nach dem Isocyanat-Polyadditionsverfahren.

EP 0 241 690 A2

BAYER AKTIENGESELLSCHAFT 5090 Leverkusen, Bayerwerk
Konzernverwaltung RP
Patentabteilung Wr/by-c

# Aliphatische Diisocyanate und ihre Verwendung zur Herstellung von Polyurethankunststoffen

Die Erfindung betrifft neue aliphatische Diisocyanate, die neben einer sterisch ungehinderten Isocyanatgruppe eine sterisch gehinderte Isocyanatgruppe aufweisen, und die Verwendung der neuen Diisocyanate bei der Herstellung von Polyurethankunststoffen.

Die in der technischen Polyurethanchemie eingesetzten aliphatischen Diisocyanate, insbesondere das Hexamethylendiisocyanat, aber auch Trimethyl-1,6-hexandiisocyanat und 2-Methyl-1,5-pentandiisocyanat eignen sich wegen ihres aliphatischen Charakters hervorragend zur Herstellung von lichtechten Polyurethankunststoffen. Diese Diisocyanate werden daher insbesondere zur Herstellung von Polyurethanlacken oder zur Herstellung von Lackpolyisocyanaten eingesetzt. Ein Nachteil dieser wichtigen aliphatischen Diisocyanate des Standes der Technik ist in dem Umstand zu sehen, daß ihre Isocyanatgruppen eine gleiche oder ähnliche Reaktivität gegenüber Isocyanatreaktiven Gruppen aufweisen, was z. B. Umsetzungen erschwert, bei denen nur eine Isocyanatgruppe zur Reaktion gebracht werden soll, z. B. die Herstellung von NCO-Präpolymeren durch Reaktion einer Isocyanatgruppe des Diisocyanate mit Hydroxylgruppen von Polyhydroxylverbindungen der aus der Polyurethanchemie bekannten Art.

Es war daher die der Erfindung zugrundeliegende Aufgabe, neue aliphatische Diisocyanate zur Verfügung zu stellen, die sich einerseits ebenso wie die bekannten aliphatischen Diisocyanate sehr gut zur Herstellung von lichtechten Polyurethankunststoffen eignen, und die andererseits Isocyanatgruppen einer ausgeprägt unterschiedlichen Reaktivität aufweisen.

Diese Aufgabe konnte durch die Bereitstellung der nachstehend näher beschriebenen erfindungsgemäßen Diisocyanate gelöst werden.

Gegenstand der Erfindung sind Diisocyanate der allgemeinen Formel

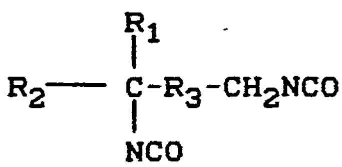

I

in welcher

$R_1$ und $R_2$ für gleiche oder verschiedene Reste stehen und Alkylgruppen mit 1 - 4 Kohlenstoffatomen bedeuten und

$R_3$ für einen zweiwertigen, gegebenenfalls verzweigten, gesättigten aliphatischen Kohlenwasserstoffrest mit 2 - 9 Kohlenstoffatomen steht.

Gegenstand der Erfindung ist auch die Verwendung der erfindungsgemäßen Diisocyanate als Isocyanatkomponente bei der Herstellung von Polyurethankunststoffen nach dem Isocyanat-Polyadditionsverfahren.

Die erfindungsgemäßen Diisocyanate können hergestellt werden, indem man ungesättigte Amine der allgemeinen Formel II oder III,

$$\begin{array}{l} R_1 \\ | \\ C{=}CH{-}R'_3{-}CH_2NH_2 \\ | \\ R_2 \end{array} \qquad \text{II}$$

$$\begin{array}{l} \quad\;\; R_1 \\ \quad\;\; | \\ H_2C{=}C{-}R_3{-}CH_2NH_2 \end{array} \qquad \text{III}$$

in welchen

$R_1$, $R_2$ und $R_3$ die obengenannte Bedeutung haben und $R'_3$ für einen gegebenenfalls verzweigten, gesättigten, 2-wertigen aliphatischen Kohlenwasserstoffrest mit 1 bis 8 Kohlenstoffatomen steht.

oder Aminoalkohole der allgemeinen Formel IV

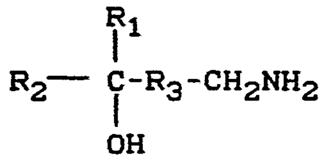

IV

in welcher $R_1$, $R_2$ und $R_3$ die oben angegebene Bedeutung haben, in einer Ritter-Reaktion mit einem Nitril der allgemeinen Formel V,

$$R\text{-}CN \qquad V$$

in der R für Wasserstoff, einen gegebenenfalls substituierten Alkyl, Cycloalkyl- oder Arylrest, vorzugsweise für Wasserstoff steht,

zu den Diaminen der allgemeinen Formel VI,

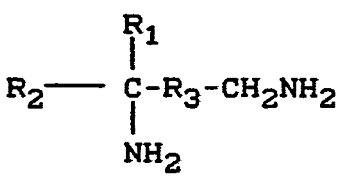

VI

in welcher $R_1$, $R_2$ und $R_3$ die oben angegebene Bedeutung haben, umsetzt.

Aus den Diaminen der allgemeinen Formel VI erhält man durch Phosgenierung die Diisocyanate der allgemeinen Formel I.

Die ungesättigten Amine der allgemeinen Formel II oder III bzw. die Aminoalkohole der allgemeinen Formel IV sind entweder bekannt oder können aus Verbindungen der allgemeinen Formel VII, VIII oder IX,

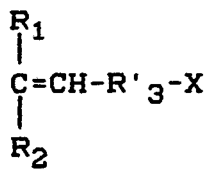

VII

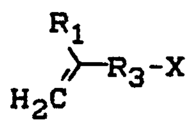

VIII

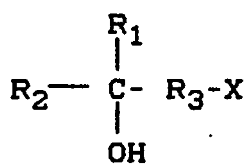

IX

in welchen $R_1$, $R_2$, $R_3$ und $R'_3$ die oben angegebene Bedeutung haben und

-X für CHO oder -CN steht,
durch reduktive Aminierung bzw. Hydrierung hergestellt werden.

Bevorzugte Ausgangsmaterialien sind solche der allgemeinen Formeln VII oder VIII wie z.B. 3-Methyl-but-2-en-nitril, 4-Methyl-pent-3-ennitril, 5-Methyl-hex-4-enal,

5-Methyl-hex-5-ennitril, 2,2,5-Trimethyl-hex-4-enal und das natürliche Citronellal. Diese Ausgangsmaterialien führen letztendlich zu den besonders bevorzugten erfindungsgemäßen Diisocyanaten, d.h. zu 1,4-Diisocyanato-4-methylpentan, 1,5-Diisocyanato-5-methylhexan, 1,6-Diisocyanato-6-methylheptan, 1,5-Diisocyanato-2,2,5-trimethylhexan bzw. zu 1,7-Diisocyanato-3,7-dimethyloctan.

Die Ritter-Reaktion der ungesättigten Amine der allgemeinen Formel II oder III bzw. der Aminoalkohole der allgemeinen Formel IV wird in Gegenwart einer starken Säure, wie Schwefelsäure, Phosphorsäure, Alkyl-oder Arylsulfonsäure, Trifluoressigsäure oder Ameisensäure durchgeführt. Bevorzugt wird Schwefelsäure verwendet. Der Wassergehalt der Säure kann zwischen 0 und 40 % betragen, vorzugsweise jedoch 15 bis 25 %. Pro Mol ungesättigtes Amin verwendet man 1 bis 20 Mol Säure, vorzugsweise 1 bis 3 Mol, besonders bevorzugt 2 Mol. Als Nitril können Blausäure, Acetonitril, Propionitril, Benzylcyanid oder Benzonitril, vorzugsweise Blausäure verwendet werden. Bezogen auf das ungesättigte Amin der allgemeinen Formel II oder III bzw. dem Aminoalkohol der allgemeinen Formel IV wird das Nitril der allgemeinen Formel V in equimolaren Mengen bis zu einem fünffachen molaren Überschuß eingesetzt. Bevorzugt verwendet man einen 1- bis 1,3-fachen molaren Überschuß. In einer bevorzugten Verfahrensweise wird das ungesättigte Amin der allgemeinen Formel II oder III bzw. der Aminoalkohol der allgemeinen Formel IV und die Blausäure gleichzeitig zu

der Säure gegeben. Die Temperatur wird während der Zugabe zwischen 0 und 70°, vorzugsweise zwischen 30 und 50°C gehalten. Die Temperatur während der Nachreaktion wird zwischen 30 und 100°, vorzugsweise zwischen 40 und 60° gehalten. Die Ritter-Reaktion kann unter Druck, vorzugsweise jedoch drucklos durchgeführt werden. Nach einer Reaktionszeit von 1 bis 15 Stunden, vorzugsweise 2 bis 5 Stunden wird das gebildete Formamid entweder sauer oder nach Zugabe einer Base wie z. B. Natronlauge alkalisch hydrolysiert und das Diamin der allgemeinen Formel VI vom alkalischen Abwasser abgetrennt.

Das zur Herstellung von 1,4-Diisocyanato-4-methylpentan einzusetzende 1,4-Diamino-4-methylpentan ist im übrigen bekannt und in der Literatur beschrieben (z.B. US-PS 2 864 863). Es kann beispielsweise durch katalytische Hydrierung von 4-Nitro-4-methyl-valeronitril hergestellt werden. Das dem Diamin zugrundeliegende 4-Nitro-4-methylvaleronitril ist ebenfalls bekannt und in der Literatur beschrieben (z.B. J. chem. Soc. 1947, Seite 1506). Es ist besonders gut durch Umsetzung von 2-Nitropropan mit Acrylsäurenitril herzustellen.

Die durch die Ritter-Reaktion oder auf anderem Wege gewonnenen Diamine der allgemeinen Formel (VI) können zur Herstellung der entsprechenden Diisocyanate durch die an sich bekannte Phosgenierungsreaktion als solche oder auch in Form ihrer Additionsverbindungen mit Chlorwasserstoff (Hydrochlorid-Phosgenierung) oder mit Kohlendioxid (Carbamat-Methode) zum Einsatz gelangen.

Die Phosgenierung erfolgt im übrigen nach an sich bekannten Methoden, wie sie beispielsweise in Liebigs Annalen der Chemie, Band 562, Jahrgang 1949, Seiten 75 bis 109, in Ullmanns Enzyklopädie der technischen Chemie, Band 14, 4. Auflage, 1977, Seiten 350 bis 354 oder in Houben-Weyl, Methoden der organischen Chemie, Band E4, 4. Auflage, 1983, Seiten 741 bis 753 beschrieben sind.

Die Phosgenierung kann kontinuierlich oder diskontinuierlich bevorzugt in Gegenwart eines organischen Lösungsmittels durchgeführt werden. Geeignete Lösungsmittel sind die für Phosgenierungen üblicherweise verwendeten Lösungsmittel, wie aliphatische, cycloaliphatische oder aromatische Kohlenwasserstoffe, Halogenkohlenwasserstoffe, Nitrokohlenwasserstoffe, aliphatisch-aromatische Ether, aromatische Ether, Carbonsäureester, Carbonsäurenitrile, Sulfone, Phosphorsäurehalogenide oder Phosphorsäureester. Als Beispiele für geeignete Lösungsmittel seien genannt: Trimethylpentan, Methylcyclohexan, Toluol, 1,2-Dichlorethan, Chlorbenzol, Chlortoluol, 1,2-Dichlorbenzol, Anisol, Diphenylether, Essigsäurebutylester, Tetramethylensulfon, Acetonitril, Phosphoroxychlorid. Beliebige Gemische der beispielhaft genannten Lösungsmittel können selbstverständlich ebenfalls eingesetzt werden.

Bei der Phosgenierung kommen im allgemeinen 2 bis 70, vorzugsweise 2 bis 40-gew.-%ige Lösungen der zu phosgenierenden Ausgangsmaterialien, bezogen auf das Gesamtgewicht der Lösungen, zum Einsatz.

Im allgemeinen wird bei der Phosgenierungsreaktion das zu phosgenierende Ausgangsmaterial mit einer 1- bis 10-fachen, vorzugsweise 1,05- bis 7-fachen stöchiometrischen Menge an Phosgen zusammengebracht. Die genannte Phosgenmenge kann in einer Portion oder auch in Teilmengen in das Reaktionsgemisch eingegeben werden. Es kann vorteilhaft sein, z.B. bei einer diskontinuierlichen Arbeitsweise, erst einen Teil der zu verwendenden Phosgenmenge in das Reaktionsgemisch einzubringen und den restlichen Anteil in weiteren Portionen oder stetig über einen längeren Zeitraum verteilt in das Reaktionsgemisch einzubringen.

Die Phosgenierungsreaktion kann nach dem bekannten Prinzip der "Kalt-Heiß-Phosgenierung" zweistufig oder nach dem Prinzip der "Heiß-Phosgenierung" einstufig erfolgen. Bei der "Kalt-Heiß-Phosgenierung" erfolgt die Umsetzung des zu phosgenierenden Ausgangsmaterials zu Beginn der Reaktion im allgemeinen bei -20 bis +40° C, vorzugsweise -10 bis +30° C und die anschließende Heißphosgenierung bei 40 bis 260° C, vorzugsweise 80 bis 230° C.

Bei dieser "Kalt-Heiß-Phosgenierung" kann der Bereich zwischen der Anfangstemperatur und der erhöhten Temperatur gleichmäßig oder in Sprüngen durchlaufen werden.

Bei der "Heiß-Phosgenierung" kommt das zu phosgenierende Ausgangsmaterial mit dem Phosgen sofort bei Temperaturen von 40 bis 260° C, vorzugsweise 80 bis 220° C in Kontakt.

Die Phosgenierung erfolgt vorzugsweise unter Normaldruck oder bei erhöhtem Druck. Der Reaktionsdruck beträgt im allgemeinen 0,9 bis 100 bar, vorzugsweise 1 bis 70 bar.

Grundsätzlich läßt sich die Phosgenierung durch Zugabe von Katalysatoren, beispielsweise Dimethylformamid, und/oder Säureakzeptoren, beispielsweise Pyridin, beschleunigen. Im allgemeinen jedoch sind die Reaktionsgeschwindigkeiten bei der Phosgenierung auch ohne Zugabe eines derartigen Katalysators ausreichend.

Die Reaktionsdauer bei der Phosgenierung ist von den angewandten Reaktionsbedingungen, insbesondere von den Reaktionstemperaturen, vom Phosgen-Überschuß, von der Verdünnung mit Lösungsmittel und von gegebenenfalls zugegebenen Katalysatoren und/oder Säureakzeptoren abhängig.

Zur Unterdrückung von eventuellen Nebenreaktionen bei der Phosgenierung kann es vorteilhaft sein, die Reaktionsdauer vergleichsweise kurz, beispielsweise auf 0,5 bis 5 Minuten einzustellen und die Reaktionstemperatur entsprechend hoch, beispielsweise auf 170 bis 220°C einzustellen. Das kann z.B. so bewerkstelligt werden, daß man kontinuierlich eine auf 130 bis 160°C vorgeheizte Lösung des Diamins in einem inerten Lösungsmittel auf eine auf 200 bis 230°C vorgeheizte Lösung der 5- bis 7-fachen stöchiometrischen Menge an Phosgen in einem inerten Lösungsmittel mittels eines Mischaggregats, beispielsweise einer Mischdüse miteinander vermischt, daß Gemisch in einem kontinuierlich durchströmten und

gegebenenfalls beheizten Reaktionsrohr bei entsprechend hoch eingestelltem Überdruck über eine mittlere Verweilzeit von 0,5 bis 5 Minuten zur Reaktion bringt und schließlich das Reaktionsgemisch durch Entspannen des Druckes rasch abkühlt.

Wenn bei der Phosgenierungsreaktion die Additionsverbindungen des Diamins mit Chlorwasserstoff oder Kohlendioxid in einem Lösungsmittel eingesetzt werden, das bei den angewandten Reaktionstemperaturen diese Additionsverbindungen nur wenig löst, so ist es im allgemeinen vorteilhaft, die Additionsverbindungen in fein verteilter Form umzusetzen. Aufgrund der großen Oberfläche können dann die Additionsverbindungen trotz der geringen Löslichkeit in annehmbaren Reaktionszeiten umgesetzt werden.

Nach Beendigung der Phosgenierungsreaktion wird das Reaktionsgemisch in an sich bekannter Weise durch Abtrennung von gasförmigen Bestandteilen (Chlorwasserstoff, überschüssiges Phosgen) und destillativer Entfernung des Lösungsmittels oder, falls ein höher als das Diisocyanat siedendes Lösungsmittel verwendet wurde, durch destillative Isolierung des Diisocyanats aufgearbeitet. Gegebenenfalls vorliegendes festes Nebenprodukt und/oder gegebenenfalls vorliegendes festes Ausgangsmaterial können durch Filtrieren oder Zentrifugieren entfernt werden.

Nach der destillativen Aufarbeitung des Reaktionsgemisches können die erfindungsgemäßen Diisocyanate der allgemeinen Formel I in reiner Form erhalten werden. Ihr NCO-Gehalt liegt im allgemeinen innerhalb des Bereichs von 20 bis 50, vorzugsweise von 35 bis 50 Gew.-%. Sie stellen wertvolle Ausgangsmaterialien zur Herstellung von Polyurethankunststoffen nach dem Isocyanat-Polyadditionsverfahren dar. Bei dieser erfindungsgemäßen Verwendung der neuen Diisocyanate, insbesondere zur Herstellung von Polyurethan-Anstrichstoffen und -Beschichtungen werden die erfindungsgemäßen Diisocyanate anstelle der bislang für diesen Verwendungszweck eingesetzten Diisocyanate mit den bekannten Reaktionspartnern zur Reaktion gebracht. (vgl. diesbezüglich beispielsweise "Kunststoff-Handbuch", Band 7, "Polyurethane" von Vieweg und Höchtlen, 2. Auflage, Carl Hanser Verlag, 1983, insbesondere Seiten 540 - 580.).

Bei der erfindungsgemäßen Verwendung der neuen Diisocyanate können diese sowohl als solche oder auch in mit Blockierungsmitteln für Isocyanatgruppen, wie z. B. ε-Caprolactam, Methyl-ethyl-ketoxim, Malonsäurediethylester oder Acetessigsäureethylester blockierter Form zum Einsatz gelangen.

Die neuen Diisocyanate eignen sich insbesondere zur Herstellung von Polyurethankunststoffen nach dem Zweistufen-Prinzip, wobei in einer ersten Stufe zunächst aus

den erfindungsgemäßen Diisocyanaten und unterschüssigen Mengen an organischen Polyhydroxylverbindungen, der in der Polyurethanchemie an sich bekannten Art freie Isocyanatgruppen aufweisende Prepolymere hergestellt werden, die anschließend in einer zweiten Reaktionsstufe mit geeigneten Kettenverlängerungsmitteln in das hochmolekulare Polyurethan überführt werden.

In den nachfolgenden Beispielen beziehen sich alle Druckangaben (Siedepunkte) auf mbar und alle Temperaturangaben ebenso wie in den vorstehenden Ausführungen auf °C.

## Beispiele

In den nachfolgenden Beispielen 1 und 2 werden die folgenden ungesättigten Amine eingesetzt:

### Ungesättigtes Amin A

Ein 10 l-Autokav wird mit 2000 g 5-Methyl-hex-4-enal, 20 g Ammoniumacetat, 200 g Raney-Cobalt und 3200 ml flüssigem Ammoniak beschickt. Man drückt 70 bar Wasserstoff auf und erwärmt unter Rühren auf 90° C, wobei man den Wasserstoffdruck bei 80 bis 100 bar hält. Nach 4 h ist die Wasserstoffaufnahme beendet. Nach dem Abkühlen wird entspannt und das Ammoniak abgedampft. Man filtriert vom Katalysator ab und destilliert i. Vak. Man erhält 1750 g 5-Methylhex-4-enylamin, $Kp._{20}$ 52 - 54° C.

### Ungesättigtes Amin B

Ein 3 l-Autoklav wird mit 900 g Citronellal, 10 g Ammoniumacetat, 100 g Raney-Cobalt und 1000 ml flüssigem Ammoniak beschickt. Die reduktive Aminierung ist bei 90° C und einem Wasserstoffdruck von 80 bis 100 bar nach 90 min beendet. Nach dem Abdampfen des Ammoniak wird vom Katalysator abfiltriert und i. Vak. destilliert. Man erhält 690 g 3,7-Dimethyloct-6-enylamin, $Kp._{10}$ 93 -97°.

Beispiel 1

a) Herstellung von 5-Methylhexan-1,5-diamin

Bei 40° werden gleichzeitig 300 ml Blausäure und 678 g des ungesättigten Amins A unter kräftigem Rühren in 1 h in 2054 g 80 % Schwefelsäure eingetropft. Man rührt 2,5 h bei 40° nach und läßt 4000 g 50 % Natronlauge zulaufen. Man erhitzt 4 h am Rückfluß, läßt bei 80° 2 l Toluol zulaufen, trennt die organische Phase bei 70 -80° ab und extrahiert die wäßrige Salzsuspension bei 70 -80° mit 1 l Toluol. Die Destillation i. Vak. ergibt 621 g 5-Methylhexan-1,5-diamin, $Kp_{20}$ 83 - 84°.

b) Herstellung von 5-Methylhexan-1,5-diisocyanat

Eine Lösung von 97,5 g 5-Methylhexan-1,5-diamin in 1500 ml Chlorbenzol wird bei 90° mit $CO_2$ gesättigt. Bei 0 - 20° läßt man eine Lösung von 450 g Phosgen in 1350 ml Chlorbenzol zulaufen. Unter Durchleiten von Phosgen erwärmt man in 2 h auf 40°, phosgeniert 5 h bei 40° und 2 h bei 130° und bläst die klare Lösung mit Stickstoff aus. Nach dem Abdestillieren des Lösungsmittels wird das Rohprodukt in Vak. destilliert. Man erhält 126 g 5-Methylhexan-1,5-diisocyanat, $Kp._{20}$ 90 - 95°.

## Beispiel 2

a) Herstellung von 3,7-Dimethyloctan-1,7-diamin

Bei 40° werden gleichzeitig 590 g des ungesättigten Amin B und 180 ml Blausäure unter kräftigem Rühren in 1 h in 1306 g 80 % Schwefelsäure eingetropft. Man rührt 3 h bei 40 ° nach und läßt 3650 g 45 % Natronlauge zulaufen, erhitzt 5 h am Rückfluß, gibt 2 l Chlorbenzol hinzu, trennt die organische Phase bei 80° ab und extrahiert die wäßrige Salzsuspension bei 70 - 80° mit 1 l Chlorbenzol. Die Destillation i. Vak. ergibt 589 g 3,7-Dimethyloctan-1,7-diamin, $Kp._{10}$ 110 - 115°.

b) Herstellung von 3,7-Dimethyloctan-1,7-diisocyanat

Eine Lösung von 50 g 3,7-Dimethyloctan-1,7-diamin in 800 ml Chlorbenzol wird bei 90° mit $CO_2$ gesättigt. Man läßt bei 0 - 20° eine Lösung von 180 g Phosgen in 500 ml andestilliertem Chlorbenzol zulaufen und erwärmt unter Durchleiten von Phosgen in 1 h auf 40°, phosgeniert 4 h bei 40° und 2 h bei 130° weiter und bläst mit $N_2$ aus. Nach dem Abdestillieren des Lösungsmittels wird das rohe Diisocyanat i. Vak. destilliert. Man erhält 58 g 3,7-Dimethyloctan-1,7-diisocyanat, $Kp_{0,2}$ 85 - 89°.

Beispiel 3 (Herstellung von 1,4-Diisocyanato-4-methylpentan; Direktphosgenierung)

In einer 6 l-Laborphosgenierapparatur wurde eine Lösung von 600 g Phosgen in 2 l wasserfreiem Chlorbenzol vorgelegt und bei 10 bis 20° C unter kräftigem Rühren im Verlauf von 1 Stunde mit einer Lösung von 116 g 1,4-Diamino-4-methylpentan in 1,5 l wasserfreiem Chlorbenzol versetzt. Anschließend wurde das Reaktionsgemisch unter weiterem Einleiten von Phosgen (ca. 100 g/h) zunächst zügig auf 60° C, dann im Verlauf von 3 Stunden zum Rückfluß (127° C) erhitzt. Schließlich wurde das Gemisch noch 1 Stunde am Rückfluß phosgeniert und dann durch Andestillieren von überschüssigem Phosgen befreit. Die trübe Lösung wurde abgekühlt und filtriert. Der abgetrennte Feststoff wurde mit Chlorbenzol und Petrolether gewaschen und im Vakuum getrocknet. Der Feststoff wurde als 1,4-Diamino-4-methylpentan-bishydrochlorid identifiziert (Ausbeute: 43 g = 23 % der Theorie bezogen auf eingesetztes Diamin). Die klare Reaktionslösung wurde durch Destillation von Chlorbenzol befreit. Die zurückbleibende bräunliche Flüssigkeit wurde im Vakuum destilliert, wobei 124 g Diisocyanat (74 % der Theorie bezogen auf eingesetztes Diamin) als farblose Flüssigkeit vom Siedepunkt 101 bis 103° C bei 13 mbar erhalten wurden.

$C_8H_{12}N_2O_2$ (168)
NCO-Gehalt ber.: 50,0 % gef.: 49,6 %

<u>Beispiel 4</u> (Herstellung von 1,4-Diisocyanato-4-methylpentan; Carbamat-Methode)

In einer 6 l-Laborphosgenierapparatur wurde eine Lösung von 116 g 1,4-Diamino-4-methylpentan in 4 l wasserfreiem 1,2-Dichlorbenzol vorgelegt. In die Lösung wurde bei Raumtemperatur unter intensivem Rühren langsam wasserfreies Kohlendioxid in Form feinverteilter Gasbläschen bis zur Sättigung eingeleitet. In die entstandene feinteilige Suspension wurden dann bei Raumtemperatur 500 g Phosgen eingeleitet. Anschließend wurde das Gemisch unter weiterem Einleiten von Phosgen (ca. 100 g/h) zuerst zügig auf 60°C, dann langsam im Verlauf von 3,5 Stunden bis zum Rückfluß (175°C) erhitzt. Nach weiteren 30 Minuten Phosgenieren am Rückfluß wurde das Reaktionsgemisch durch Andestillieren von überschüssigem Phosgen befreit, dann abgekühlt und durch Filtrieren von Feststoffanteilen befreit (7,6 g = 4 % der Theorie 1,4-Diamino-4-methylpenbtan-bis-hydrochlorid). Aus der klaren Reaktionslösung wurde das Lösungsmittel im Vakuum über eine trennwirksame Kolonne abdestilliert. Das zurückbleibende dunkel gefärbte Rohisocyanat wurde im Vakuum destilliert, wobei 158 g farbloses Destillat mit einem NCO-Gehalt von 48,7 % erhalten wurden. Nach gaschromatographischer Analyse bestand das Destillat zu 94,8 % aus 1,4-Diisocyanato-4-methylpentan.

Ausbeute: 89 % der Theorie bezogen auf eingesetztes Diamin

<u>Beispiel 5</u> (Herstellung von 1,4-Diisocyanato-4-methylpentan; Hydrochlorid-Phosgenierung)

In einer 6 l-Laborphosgenierungsapparatur wurde eine Lösung von 116 g 1,4-Diamino-4-methylpentan in 4 l wasserfreiem 1,2-Dichlorbenzol vorgelegt. In die Lösung wurden bei Raumtemperatur unter Kühlen und intensivem Rühren langsam 73 g Chlorwasserstoff in Form feinverteilter Gasbläschen eingeleitet. Die entstandene feinteilige Suspension wurde dann unter Einleiten von Phosgen (ca. 150 g/h) zügig bis zum Rückfluß (175°C) erhitzt und dann 4 Stunden am Rückfluß phosgeniert. Anschließend wurde das Reaktionsgemisch durch Andestillieren von überschüssigem Phosgen befreit, dann abgekühlt und durch Filtration von Feststoffanteilen befreit (34 g = 18 % der Theorie 1,4-Diamino-4-methylpentan-bis-hydrochlorid, bezogen auf eingesetztes Diamin). Aus der klaren Reaktionslösung wurde dann das Lösungsmittel im Vakuum über eine trennwirksame Kolonne abdestilliert. Das zurückbleibende dunkel gefärbte Rohprodukt wurde im Vakuum destilliert, wobei 129 g farbloses Destillat mit einem NCO-Gehalt von 47,6 % erhalten wurden. Nach gaschromatographischer Analyse bestand das Destillat zu 89,7 % aus 1,4-Diisocyanato-4-methylpentan.

Ausbeute: 69 % der Theorie bezogen auf eingesetztes Diamin.

## Patentansprüche

1. Diisocyanate der allgemeinen Formel

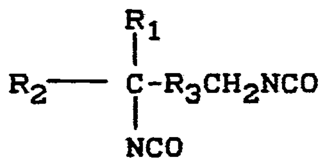

I

in welcher

$R_1$ und $R_2$ für gleiche oder verschiedene Reste stehen und Alkylgruppen mit 1 - 4 Kohlenstoffatomen bedeuten und

$R_3$ für einen zweiwertigen, gegebenenfalls verzweigten, gesättigten aliphatischen Kohlenwasserstoffrest mit 2 - 9 Kohlenstoffatomen steht.

2. 1,4-Diisocyanato-4-methylpentan.

3. 1,5-Diisocyanato-5-methylhexan.

4. 1,6-Diisocyanato-6-methylheptan.

5. 1,5-Diisocyanato-2,2,5-trimethyl-hexan.

6. 1,7-Diisocyanato-3,7-dimethyl-octan.

7. Verwendung der Diisocyanate gemäß Anspruch 1 bis 6 als Isocyanatkomponente bei der Herstellung von Polyurethankunststoffen nach dem Isocyanat-Polyadditionsverfahren.